# EUROPÄISCHE PATENTSCHRIFT

(11) **EP 0 457 866 B1**
(45) Veröffentlichungstag und Bekanntmachung des Hinweises auf die Patenterteilung: **06.04.1994**
(21) Anmeldenummer: 90917290.0
(22) Anmeldetag: 05.12.1990
(51) Int. Cl.: A61F 2/36

(54) **ADAPTIERBARER SCHAFT FÜR EINE ENDOPROTHESE**
ADAPTABLE STEM FOR AN ENDOPROSTHESIS
TIGE ADAPTABLE POUR ENDOPROTHESES

(30) Priorität: 07.12.1989 CH 4399/89
(43) Veröffentlichungstag der Anmeldung: 27.11.1991
(73) Patentinhaber: AO-FORSCHUNGSINSTITUT DAVOS, 7270 Davos-Platz (CH)
(72) Erfinder: KLAUE, Kaj, CH-3028 Spiegel (CH); CHAREIRE, Jean, Louis, F-92300 Levallois (FR)
(74) Vertreter: Lusuardi, Werther Giovanni, Dr.
(86) Internationale Anmeldenummer: CH9000281
(87) Internationale Veröffentlichungsnummer: WO9108720

(56) Entgegenhaltungen:
- WO-A-82/03323
- FR-A- 2 629 337
- US-A- 3 893 196
- US-A- 4 888 022
- US-A- 4 888 024

## Beschreibung

Die Erfindung betrifft einen Schaft für eine in einem Knochen zu verankernde Endoprothese und dessen Verwendung zur Verankerung von Gelenkendoprothesen, im speziellen für Hüftprothesen.

Schaftprothesen dieser Art weisen in der Regel einen steif ausgebildeten, stielartigen Abschnitt aus Metall oder Kompositmaterial auf, welcher mit oder ohne Knochenzement in die präparierte Knochenraumhöhle beispielsweise des Femur fixiert wird.

In den sogenannten zementierten Prothesen, umgibt der Zement die Innenfläche des Röhrenknochens. Die Nachteile dieser bekannten Implantationstechnik sind einerseits die exotherme Reaktion des polymerisierenden Zements, andererseits füllt der Zement sämtliche medullären Kavitäten aus, ohne die Härte deren Wände zu berücksichtigen; dies hat zur Folge, dass der Kraftschluss Prothese-Knochen über eine sehr heterogene Zwischenschicht erfolgt, zumindest was den mechanischen Widerstand betrifft. Wenn die harten Zonen des Knochens in diesem "interface" zu selten sind, kann es schnell zu einer Lockerung kommen. Es ist bekannt, dass Mikrobewegungen sehr schnell (Monate bis einige Jahre) zwischen Prothese und Knochen auftreten, dies lange bevor sie klinisch relevant sind. Mikrobewegungen ergeben jedoch eine progressive Knochenresorption, die dann später klinisch und radiologisch evident wird. Der Zement ist im besten Fall ein bioinertes Material ohne biologische Affinität zu den Knochenzellen. Es ergibt sich daher immer eine sogenannte Fremdkörpermembran, die zumindest auf der Prothesenseite keinen Halt findet.

In den sogenannten "zementlosen" Prothesen, versucht man durch ein Verklemmen der Prothese, eine bewegungsfreie Fixation und Inkorporation zu erreichen. Es ist klar, dass die obligate Inkongruenz Prothese-Knochen punktförmige Kontaktstellen mit lokalen Überbelastungen mit sich bringt. Wenn diese lokalen Stellen zyklisch stark belastet werden, was unter funktionellen Bedingungen höchst wahrscheinlich ist, kann es zur medullären Knochenresorption und periostaler Apposition kommen, wie dies in Untersuchungen am Tier gezeigt werden konnte, welche schliesslich auch zur Lockerung führen. Andererseits sind als Vorteile zu nennen: die ausbleibende Überhitzung und eine verbesserte Affinität zum Knochen, die durch verschiedene Werkstoffe (Titanplasma, Hydroxyapatit, Carbon) möglich ist und schliesslich die mögliche Verbesserung der Extraktionsmöglichkeit der Prothese.

Aus der EP-A1 0 323 800 (Mecron) ist weiter ein implantierbarer Prothesenschaft mit einer biokompatiblen, elastischen, membranartigen Kunststoff-Umhüllung bekannt. Da diese vorbekannte Umhüllung eine definierte Wandstärke aufweist, kann damit keine wirklich befriedigende Anpassung an die Knochenmarkhöhle erzielt werden. Aber auch die Biokompatibilität der Umhüllung kann zumeist nicht ausgenützt werden, da zur Erzielung einer sicheren Verankerung nach wie vor Knochenzement eingesetzt werden muss.

Die EP-A1 0 328 848 (Sulzer) offenbart eine mit dem Prothesenschaft nicht verbundene, d.h. separat zu implantierende, netzartige Verstärkung. Die mehrlappig in Form eines "Blumenkelches" ausgebildete Verstärkung soll als Trennschicht zwischen dem Knochenzement und der Markraumhöhle dienen. Da es sich bei dieser vorbekannten Verstärkung um ein "offenes", d.h. nicht druckdichtes System handelt, kann der als Füllmaterial zwischen Prothesenschaft und Verstärkung eingespritzte Knochenzement nur eine geringe Druckwirkung auf die einzelnen Lappen der Verstärkung ausüben, so dass auch die Adaptationsfähigkeit der Verstärkung an die unregelmässig ausgebildete Markraumhöhle sehr beschränkt ist. Von besonderem Nachteil ist jedoch der Durchtritt von Knochenzementmaterial zwischen den einzelnen Lappen der Verstärkung in die Markraumhöhle, sowie nach proximal aus der Markraumhöhle hinaus, weil dadurch wiederum der unerwünschte Kontakt des Knochenzementmaterials mit dem menschlichen Gewebe erfolgt.

Schliesslich offenbart die gattungsbilbende FR-A-2629337 eine Hüftgelenk-Endoprothese, deren Femurkomponente mit einer weichen, deformierbaren Hülle umgeben ist, welche durch einen offenen Kanal mit einer aushärtbaren Masse gefüllt werden kann. Nachteilig bei dieser Ausführung ist die offene Ausführung des Kanals, welche keinen permanenten Druckaufbau in der Hülle erlaubt. Bei der vorzugsweise verwendeten, selbsthärtenden Knochenzementmasse ist aber ein permanenter Druckaufbau gar nicht notwendig. Aus dem gleichen Grunde ist bei dieser bekannten Femurkomponente auch bloss eine 1-kammerige Ausführung vorgesehen.

Hier will die Erfindung Abhilfe schaffen. Der Erfindung liegt die Aufgabe zugrunde, einen Schaft für eine in einem Röhrenknochen zu verankernde Endoprothese zu schaffen, welcher die Vorteile der zementierten und der zementlosen Prothese vereinigt und die erwähnten Nachteile überwindet, indem die "mobile", biologisch aktive, sich umbauende Knochenwand durch die kontinuierlich morphologische Adaptabilität des dynamisch expandierbaren Schaftes stets mit letzterem in einem bioaffinen Kontakt bleibt.

Die Erfindung löst die gestellte Aufgabe mit einem Schaft, welcher die Merkmale des Anspruchs 1 und einer Verwendung des Schaftes, welche die Merkmale des Anspruchs 21 aufweist.

Die durch die Erfindung erreichten Vorteile sind im wesentlichen darin zu sehen, dass dank des erfindungsgemässen Schaftes eine getrennte Lösung des Problems der Bio-Affinität und des mechanischen Kraftschlusses ermöglicht wird.
Der rigide Schaft der Endoprothese wird mechanisch zum Knochen mit einem Füllmaterial verbunden. Zusätzlich ist dieses Material jedoch vom Knochen durch die Wand einer dichten Kammer getrennt. Somit steht eine breite Palette von Möglichkeiten zur Verfügung, die Aussenseite (Knochenseite) dieser Kammer so biokompatibel (bioaffin) wie möglich zu gestalten.

Der erfindungsgemässe Prothesenschaft kann im leeren Zustand, d.h. mit ungefüllter Kammer welche eng am Schaft anliegt, leicht in die überdimensionierte Markhöhle des Knochen eingeführt werden. Danach wird die Kammer mit Füllmaterial expandiert um einen möglichst guten Kammer/Knochen-Kontakt zu realisieren.

Da der sich in der Kammer ausbildende hydrostatische Druck gleichmässig verteilt ist der Anpressdruck gegen die Innenwand des Knochens ebenfalls homogen. Dieser Druck kann auch unter Umständen mit einem lokal angebrachten Manometer dauernd überprüft werden. Um dem Druck standzuhalten, der sich an den "exponierten" Stellen ausbilden kann (Kragen und Spitze des Prothesenschaftes) können die Wände der Kammer an diesen Stellen besonders zugsteif hergestellt werden.

Anderorts ist die radiale Steifigkeit der Kammer minimal um die Expansion zu begünstigen. Die Werkstoffe, die sich für die Kammer eignen sind: für niedrige Einspritzdrücke, Silikon oder Polyurethan (dieses Material wird im speziellen in der Herzprothetik angewandt und hat sich dort als sehr biokompatibel und thrombosehemmend erwiesen) und für hohe Einspritzdrücke, eine Textilwand, die mit den gleichen Elastomeren imprägniert ist.

Vorteilhafterweise weist diese Textilwand die folgenden Eigenschaften auf:
- Geflecht aus zwei gegeneinander gekreuzten Serien von Fasern.
- Anwinkelung der Fasern ca. 45 Grad zur Knochenachse um eine radiale Extension zu begünstigen.
- biokompatible Fasern mit hoher Festigkeit wie Karbonfasern, Polyester, Polyamid oder weniger biokompatible Fasern, soweit sie sehr gut umhüllt sind.

Um die Spannung der Kammer bei der Füllung mit Füllmaterial klein zu halten, kann dessen Querschnitts-Perimeter grösser als der des Prothesenschaftes sein. Die Stärke der Wand sollte kleiner als 0,5 mm, vorzugsweise kleiner als 0,25 mm sein.

Die Wand der Kammer des erfindungsgemässen Schaftes ist vorzugsweise mit einem "bioaffinen" Werkstoff wie Hydroxyapatit oder Karbon beschichtet. Die Herstellung einer solchen membranartigen Kammer erfolgt durch:
- Verklebung von Hydroxyapatit oder Carbonpulver mit einem biokompatiblem Kleber oder "Inkrustation" nach Erwärmung der Membran und Einpressen des Pulvers.
- Verkleben einer Textilwand (siehe oben)
- Anwendung einer "Samtwand" aus Karbon wobei die Grundplatte der Samthaare mit den genannten Elastomeren imprägniert ist. Der Grund der Samtkonstruktion ist die erhöhte Kontaktfläche mit dem Knochen. Knochen-Samt bietet eine enorme Zugfestigkeit an, selbst wenn der Gesamtquerschnitt der Haare des Samts nicht mehr als 5 bis 10% der Gesamtfläche ausmachen.

Das eingespritzte Füllmaterial kann zu folgenden Werkstoffen gehören:

### A) Thermostabiles Füllmaterial

Solche Materialien sind beispielsweise 2-Komponenten-Werkstoffe, die sich nach Polymerisation und unter Wärmeeinfluss nicht mehr verformen.
Bei Verwendung eines thermostabilen Werkstoffes, sind folgende Vorteile gegenüber der üblichen "zementierten" Prothese vorhanden:
Verminderte Thermonekrose des Knochens wegen der wärmeisolierenden Membran.
Frei wählbare Viskosität des Materials (dieses kann flüssig sein).

### B) Thermoplastisches Füllmaterial:

Es handelt sich dabei um steife Werkstoffe, die durch Erwärmen verformbar sind.
Bei Verwendung eines Thermoplastes ist es möglich die Dynamik des Knochenumbaus mit einer kontinuierlichen Adaptation des prothetischen Materials zu kompensieren.
Mechanisch-dynamisch verhält sich der Thermoplast wie ein fester, rigider Werkstoff der die funktionellen Kräfte voll überträgt. Quasi-statisch jedoch fliesst ein leicht unter hydrostatischen Druck gesetzter Thermoplast, der diesen Druck integral und homogen auf die Kontaktfläche zum Knochen überträgt. Dies ergibt somit immer eine maximale Applikationsfläche Prothese-Knochen ("interface") mit einem minimalen Druck.
Die Prothese ist konisch im Schaft implantiert wobei die Belastung durch Schwerkraft und Muskelzug eine ständige Verkeilung (Eindringen der Prothese) hervorruft. Eine "starre" Prothese tendiert in diesem Fall unvermeidlich zur Lockerung wegen des Knochenumbaus.
Der Thermoplast adaptiert sich hier an die medulläre Fläche des sich umbauenden Knochens und kann somit das Auftreten von Mikrobewegungen verhindern. Um der Dynamik dieses biologischen Vorgangs zu folgen, und eine Lockerung zu verhindern, braucht es eine homogene grossflächige Kraft-Verteilung, die eventuell durch einen zusätzlichen Nachschub an Thermoplast zu unterstützen ist. Dazu kann ein zusätzlicher mit der Kammer in Verbindung stehender Vorratsbehälter mit vorgespanntem Thermoplast dienen.
Der Thermoplast sollte deshalb den folgenden Kriterien entsprechen:
1. Seine Viskosität muss tief genug sein, um:
   a) eine problemlose und rasche Injektion zu erlauben ohne:
      - zu hohe Temperatur
      - zu hohem Druck
      b) eine Adaptation seiner Form bei Körpertemperatur zu erlauben.
2. Seine Viskosität muss bei Körpertemperatur hoch genug sein um einen zuverlässigen Kraftschluss wie ein rigider Werkstoff zu gewährleisten und sämtliche dynamische Kräfte aufzufangen.
3. Er sollte eine gute Biokompatibilität aufweisen (bei z.B. möglicher Verletzung der Membran).

Um die gewünschte Viskosität zu erreichen, kann man einem eher wenig viskösen Thermoplast ein festes, biokompatibles Pulver oder Fasern beimischen, z.B. Kohlenstoff, was zur Folge hat, dass sich die Viskosität erhöht. Ein ähnliches Resultat kann mit einem Thermoplast erreicht werden, bei welchem der Phasenwechsel hart-flüssig bei 37 Grad zu steil verläuft. Als praktische Beispiele für Thermoplasten, deren Viskosität den obengenannten Kriterien folgen kann man Paraffin und Stearin nennen. Eine gute Biokompatibilität weisen auch die Silikone in deren nicht elastomeren Form auf. Silikone verwandeln sich von viskösen Körpern zu Elastomeren in Anwesenheit von Wasser. Diese Eigenschaft kann im Fall einer Anwendung als thermoplastisches prothetisches Füllelement nützlich sein, falls ein Leck in der periprothetischen Kammer auftritt: Sobald das Silikon aus der Kammer austritt "koaguliert" es in Kontakt mit physiologischer Flüssigkeit und bildet als Elastomer ein "automatisches" Verschlussmaterial für das Leck.

Bei Verwendung eines thermostabilen Zementes als Füllmaterial ist man was die periprothetische Kammer betrifft, in den Dimensionen der letzteren nicht limitiert. Wenn jedoch ein thermoplastisches Füllmaterial angewendet wird, so "schwimmt" der rigide Schaft der Endoprothese in einem (langsam) fliessenden Medium. Tendieren somit die wirkenden resultierenden Kräfte zu einer bevorzugten Richtung, so schwenkt die Prothese langsam bis zum festen Prothese-Knochen-Kontakt.

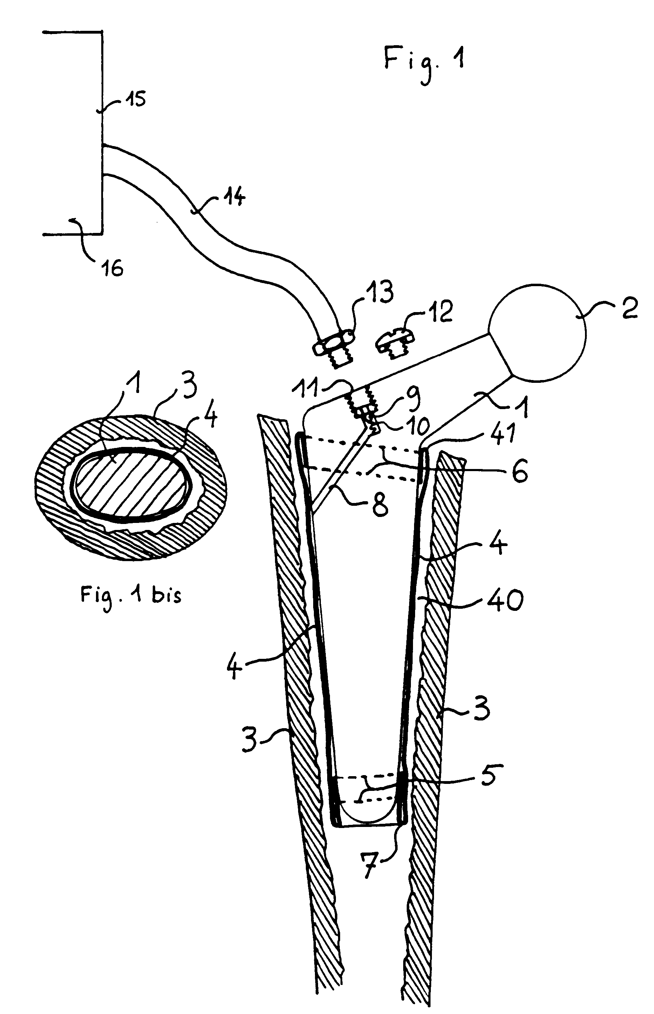

Verschiedene Ausführungsbeispiele der Erfindung, welche zugleich das Funktionsprinzip erläutern, sind in der Zeichnung dargestellt und werden im folgenden näher beschrieben. Hierbei Gallen die in den Figuren 1, 1 bis, 2, 2 bis, 7 und 7 bis dargestellten Schäfte aufgrund ihner ein teiligen Kammern nicht in den Schutzbereich des Anspruchs 1.
Fig. 1 stellt einen Längsschnitt durch einen Hüftendoprothesenschaft dar mit einer periprothetisch angeordneten Einzelkammer im leeren Zustand unmittelbar nach Einführung in die Knochenmarkhöhle;
Fig. 1bis stellt einen Querschnitt im Mittelabschnitt des Schaftes gemäss Fig. 1 dar;
Fig. 2 stellt einen Längsschnitt durch einen Hüftendoprothesenschaft gemäss Fig. 1 mit gefüllter Kammer dar;

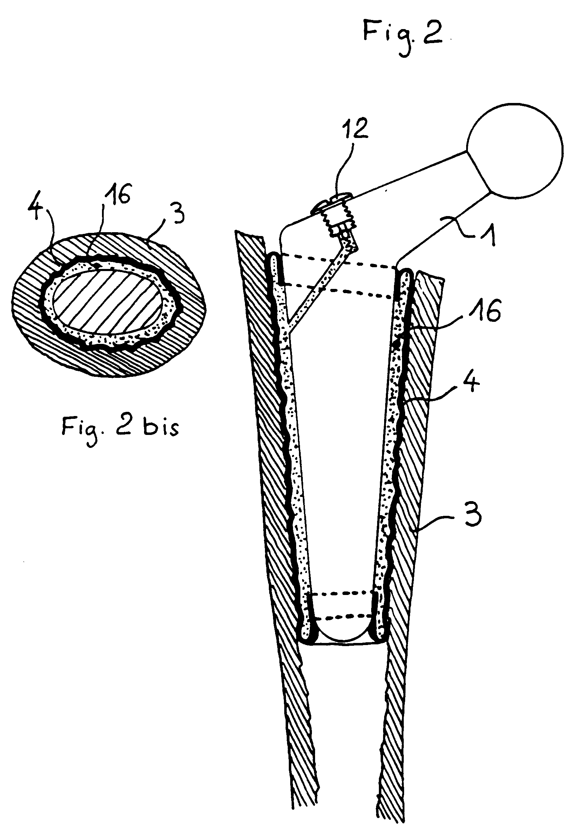

Fig. 2bis stellt einen Querschnitt im Mittelabschnitt des Schaftes gemäss Fig. 2 dar;

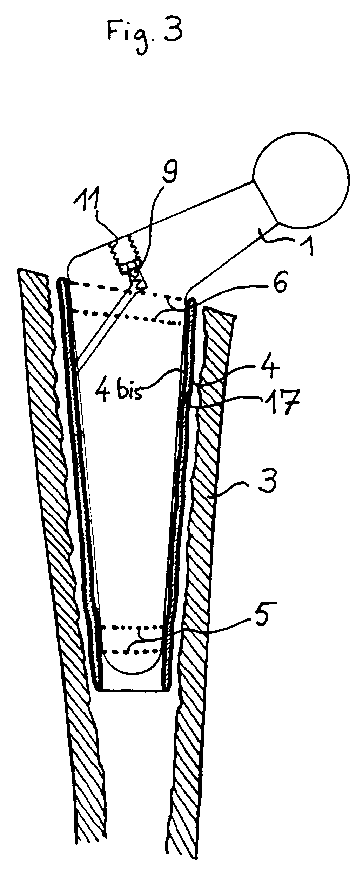

Fig. 3 stellt einen Längsschnitt durch einen Schaft gemäss der Erfindung im leeren Zustand dar mit einer schaftnahen und einer knochennahen Kammer;

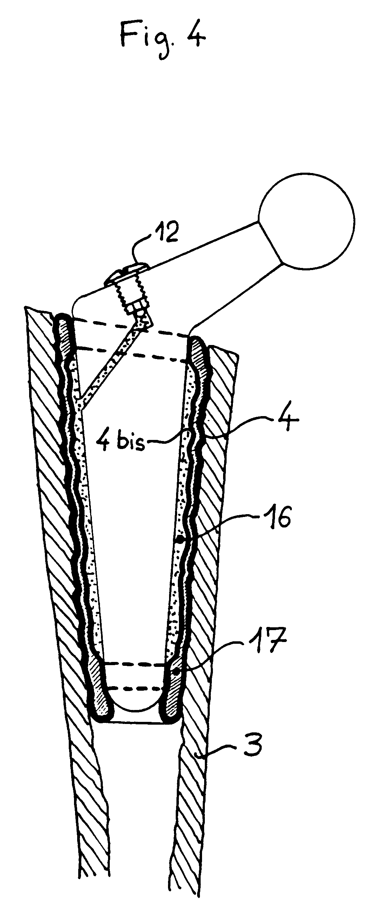

Fig. 4 stellt einen Längsschnitt durch einen Hüftendoprothesenschaft gemäss Fig. 3 mit gefüllter Kammer dar;

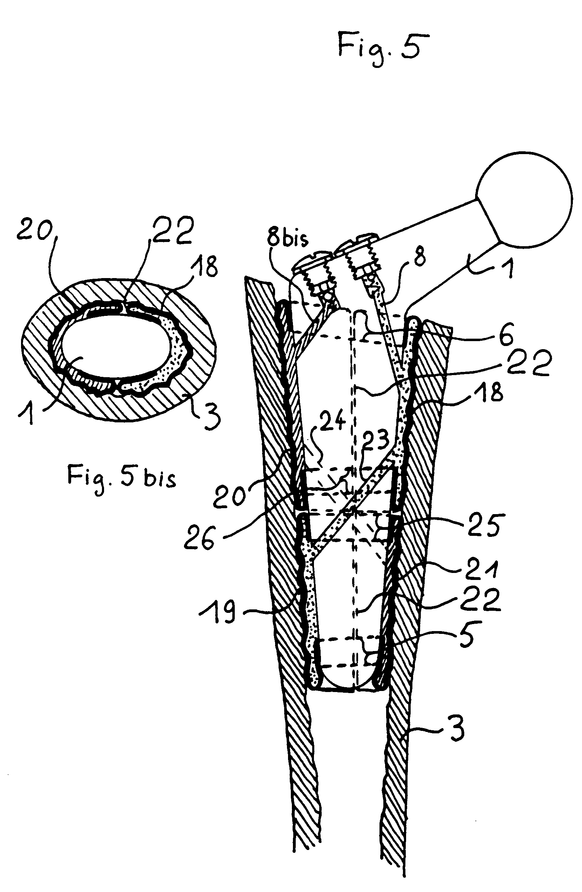

Fig. 5 stellt einen Längsschnitt durch einen Schaft gemäss der Erfindung mit vier Kammern dar;
Fig. 5bis stellt einen Querschnitt im Mittelabschnitt des Schaftes gemäss Fig. 5 dar;

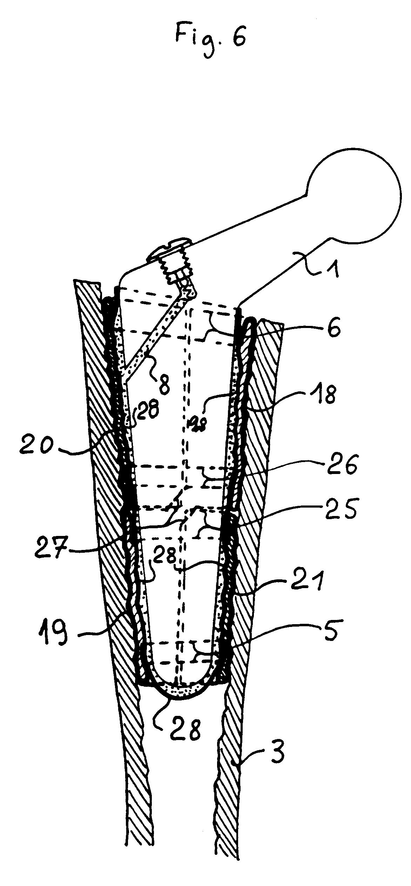

Fig. 6 stellt einen Längsschnitt durch einen Schaft gemäss Fig. 5 dar mit einer zusätzlichen, den Prothesenschaft umhüllenden Kammer;

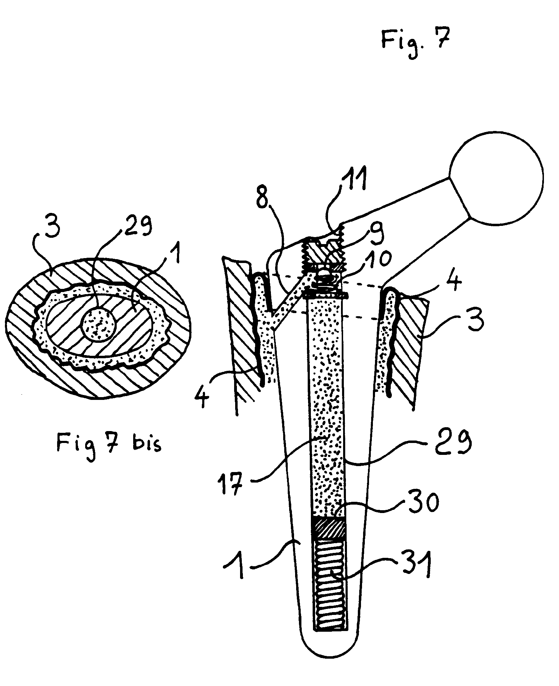

Fig. 7 stellt einen Längsschnitt durch einen Schaft mit einem Füllmaterial-Reservoir dar;
Fig. 7bis stellt einen Querschnitt im Mittelabschnitt des Schaftes gemäss Fig. 7 dar;

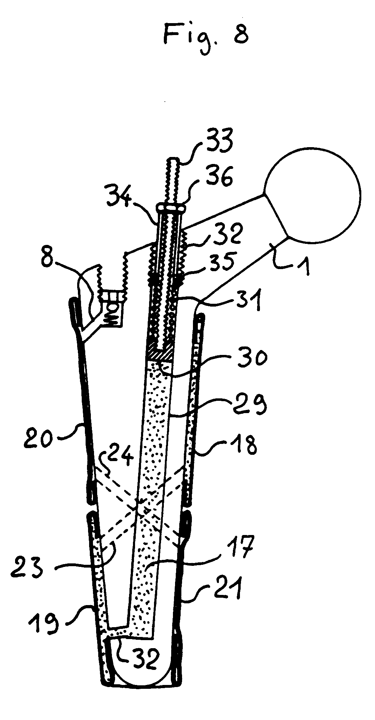

Fig. 8 stellt einen Längsschnitt durch einen Schaft gemäss der Erfindung mit einem vorspannbaren Füllmaterial-Reservoir im ungespannten Zustand dar;

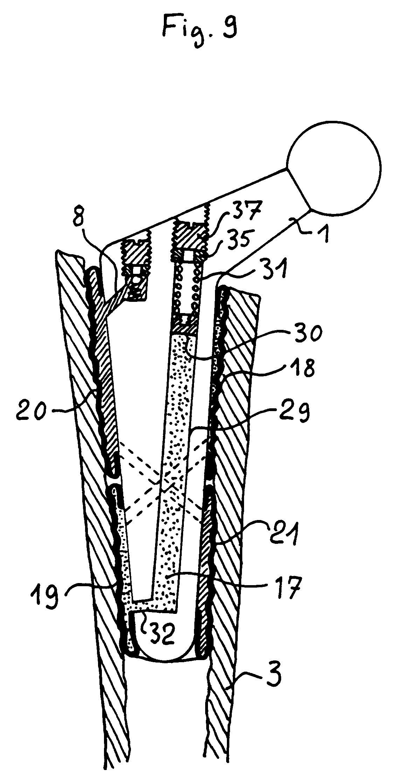

Fig. 9 stellt einen Längsschnitt durch einen Schaft gemäss der Fig. 8 im gespannten Zustand dar;

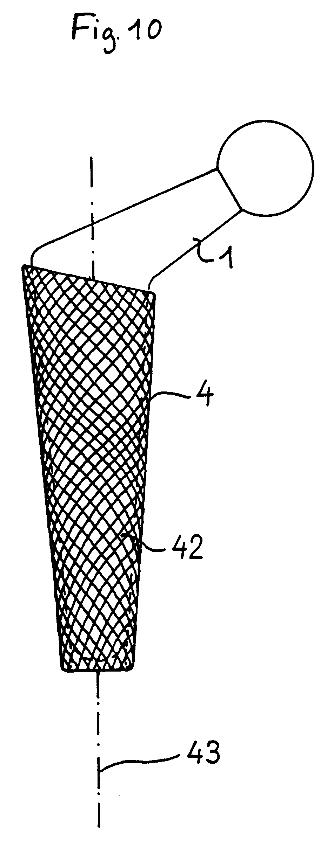

Fig. 10 stellt eine perspektivische Ansicht eines Schaft gemäss der Erfindung dar, deren Kammer aus einem Geflecht besteht; und

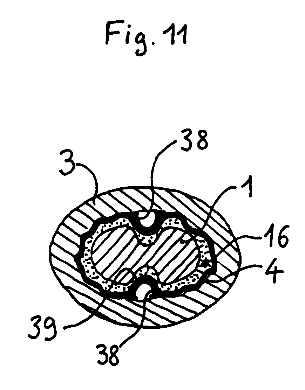

Fig. 11 stellt einen Querschnitt im Mittelabschnitt des Schaftes gemäss der Erfindung dar, dessen Kammer von aussen mit einer Schiene versehen ist, welche der Längsvaskularisation dient.

Bei der in Fig. 1 und 1bis dargestellten Ausführung ist ein rigider Prothesenschaft 1 mit Gelenkkopf 2 vollständig von einer leeren Kammer 4 periprothetisch umgeben. Die Kammer 4 besteht aus einem flexiblen Textilmaterial aus Silikon oder Polyurethan, welche längs einer distalen Befestigungszone 5 und einer proximalen Befestigungszone 6 mit dem Prothesenschaft 1 druckdicht verklebt ist. Im Schulterbereich des Prothesenschafts 1 ist ein Einwegventil 9 mit Feder 10 vorgesehen, welches über einen Kanal 8 mit der Kammer 4 kommuniziert. Das Einwegventil 9 kann über ein Gewindeloch 9 mit einem Stutzen 12 druckdicht abgeschlossen werden. Der nach Einführung des Prothesenschafts 1 mit leerer Kammer 4 in die Markhöhle des Knochens 3 bestehende, in Fig. 1 dargestellte Hohlraum 40 zwischen Aussenwand der Kammer 4 und Knochen 3 wird nun durch Expansion der Kammer 4 geschlossen. Dazu wird nach Entfernung des Stutzens 12 und Einschrauben des Einfüllstutzens 13 in das Gewindeloch 11 aus dem Füllstoffbehälter 15 über den Einfüllschlauch 14 Füllmaterial 16 durch das Einwegventil 9 via Kanal 8 in die Kammer 4 gepumpt, welche sich dadurch radial ausweitet, so dass sich - wie in Fig. 2 und 2bis ein inniger Kontakt zwischen Knochen 3 und Aussenwand der Kammer 4 ergibt.
Das Einfüllen des flüssigen oder zähflüssigen Füllmaterials 16 kann auch mittels einer Spritze erfolgen, die einen Druck von 10 bar entwickeln sollte. Der Einspritzdruck ist von der Viskosität des Füllmaterials 16 abhängig.

Bei dieser Ausführungsform (Fig. 1/2) können sowohl thermoplastische als auch thermostabile Füllmaterialien verwendet werden.
Die textile Wand der Kammer 4 ist im Bereich der distalen und proximalen Befestigungszonen 5 und 6 derart mit dem Prothesenschaft 1 verklebt, dass letzterer auch im definitiv implantierten Zustand in einem gewissen Umfang axial bewegbar bleibt, ohne dass dadurch der erzielte innige Kontakt zwischen Kammer 4 und Knochen 3 verloren geht. Diese auch als "Rollbandeffekt" bezeichnete Eigenschaft der Kammer 4, ist von einiger Wichtigkeit, da man postoperativ häufig ein "Einsinken" oder "Setzen" des Prothesenschaftes in die Markraumhöhle beobachten kann. Der "Rollbandeffekt" kann wie in den Fig. 1 und 2 dargestellt, dadurch erreicht werden, dass im distalen und proximalen Bereich je ein zirkulärer Falz 7, bzw. 41 in der Wandung der Kammer 4 vorgesehen ist.

In den Fig. 3 und 4 ist eine bevorzugte Ausführungsform des erfindungsgemässen Schaftes dargestellt, welche einen Prothesenschaft 1 mit einer Doppelkammer umfasst, welche aus einer äusseren, knochennahen Kammer 4 und einer inneren, prothesennahen Kammer 4bis besteht.
Die Implantation dieses Prothesenschaftes 1 mit Doppelkammer erfolgt derart, dass man den Prothesenschaft 1 im leeren Zustand, d.h. mit leeren Kammern 4 und 4bis, in die Markraumhöhle einführt (dieser Zustand ist in Fig. 3 dargestellt). Hierauf wird die innere, prothesennahe Kammer 4bis in situ durch Einspritzen eines thermostabilen Zementes 16 durch das Einwegventil 9 hindurch gefüllt. Danach wird die äussere, knochennahe Kammer 4 mit einem Thermoplast 17 nach leichter Erwärmung und eventuellem Nachfüllen unter Druck gestellt (dieser Endzustand ist in Fig. 4 dargestellt). Somit ist die äussere Kammer 4 auf einen Bruchteil eines Millimeters verjüngt worden.
Die Prothesenentfernung bei einem allfällig notwendig werdenden Prothesenwechsel geschieht einfach durch spezifische Erwärmung des thermoplastischen Materials.

In den Fig. 5/5bis ist eine weitere bevorzugte Variante des erfindungsgemässen Schaftes dargestellt, welche spezifisch die Schwenkgefahr einer Prothese zu vermeiden hilft. Bei dieser Ausführungsform sind insgesamt vier Kammern 18,19,20,21 vorgesehen, wobei jeweils zwei periprothetisch angeordnete Kammern 18,19 und 20,21 druckdicht miteinander verbunden sind. Die vier Kammern 18,19,20,21 berühren sich längs der Grenzlinie 22 in der Sagittalebene des Prothesenschaftes. Die Befestigung der Kammern am Prothesenschaft ist prinzipiell gleich wie in der in Fig. 1 dargestellten Ausführungsform, doch sind neben der distalen Befestigungszone 5 und proximalen Befestigungszone 6 bei dieser vierkammerigen Ausführungsform noch zusätzlich zentrale Befestigungszonen 25 und 26 und entlang der Grenzlinie 22 vorgesehen.
Somit sind die beiden proximal-medial und distal-lateral gelegenen Kammern 18 und 19 praktisch spezifisch durch die varisierenden oder Schwenkmomenten beansprucht und die zwei anderen proximal-lateral und distal-medial gelegenen Kammern 20 und 21 durch die reinen Einstauchkräfte. Die proximal-mediale Kammer 18 ist über den Verbindungskanal 23 mit der distal-lateralen Kammer 19 verbunden. Beide Kammern 18,19 können über den Einspritzkanal 8 mit Füllmaterial expandiert werden. Die proximal-laterale Kammer 20 ist über den Verbindungskanal 24 mit der distal-medialen Kammer 21 verbunden. Diese beiden Kammer 20,21 können über den Einspritzkanal 8bis mit Füllmaterial expandiert werden.
Statt der in Fig. 5 dargestellten internen Verbindungskanäle 23 und 24 können auch extern an der Prothesenoberfläche verlaufende Verbindungsgänge 27 zwischen den Kammern vorgesehen sein, wie dies bei der Ausführungsform gemäss Fig. 6 realisiert ist.

Eine komplexe Form des in den Fig. 5 und 6 dargestellten Mehrkammerprinzips lässt sich mit einer Prothese erreichen, die eine Mehrzahl von Kammern aufweist, welche je zu zweien durch die Prothese hindurch in Verbindung stehen. Diese Verbindung dient dazu den hydrostatischen Druck auszugleichen. Die Mehrzahl der Kammern, je nach Verflechtung der Verbindungsgänge, erlaubt eine Zentrierung der Prothese mit einer bewegungsfreien "interface" Knochen-Prothese.

Bei der in Fig. 6 dargestellten, weiteren Ausführungsform des erfindungsgemässen Schaftes handelt es sich um eine Modifikation des Schaftes gemäss Fig. 5, bei dem zusätzlich zu den vier Kammern 18,19,20,21 eine weitere den Schaft vollkommen umhüllende Kammer 28 vorgesehen ist, welche die vier Kammern 18,19,20,21 von der Prothese trennt. Diese umhüllende Kammer 28 kann nach erfolgter Einführung des Schaftes in die Markraumhöhle mit einem thermostabilen Zement gefüllt werden, so dass eine ähnliche Wirkung wie bei der in den Fig. 3 und 4 beschriebenen Ausführungsform erreicht werden kann.

In den Fig. 8 bis 9 sind verschiedene Ausführungsformen des erfindungsgemässen Schaftes dargestellt bei denen ein thermoplastisches Füllmaterial 17 Verwendung findet.
Der in Fig. 7/7bis dargestellte Schaft weist ein internes Reservoir 29 für den Thermoplasten 17 auf, welcher mittels der auf den Kolben 30 wirkenden Feder 31 vorgespannt ist. Damit ist es möglich den in der Kammer 4 herrschenden Druck aufrecht zu erhalten, der wegen des Knochenumbaus (Knochenresorption) sinken kann. Je nach Bedarf kann somit Thermoplast 17 aus dem Reservoir 29 durch das Einwegventil 9 mit Feder 10 via Einspritzkanal 8 in die Kammer 4 nachgeschoben werden.

In Fig. 8 ist die "gesicherte" Position der Feder 31 beim Einführen der Prothese in die Markraumhöhle dargestellt. In dieser Position ist das thermoplastische Material 17 noch nicht vorgespannt. Entfernt man nun den Gewindestab 33, die Mutter 36 sowie die Vorspannhülse 34, so wird die Feder 31 "geladen" und der Kolben 30 drückt gegen das thermoplastische Material 17, wie dies in Fig. 9 dargestellt ist. Dies erfolgt nachdem die Prothese in ihre definitive Position eingeführt worden ist.

Fig. 10 zeigt die Aussenseite der Kammer 4 eines erfindungsgemässen Prothesenschaftes 1, welche aus einem Geflecht aus zwei gegeneinander gekreuzten Serien von Fasern 42 besteht, welche eine Neigung von etwa 45° zur Schaftachse 43 aufweisen.

In Fig. 11 ist ein erfindungsgemässer Schaft dargestellt mit einem einfachen Zusatz zur periprothetischen Kammer 4. Dieser Zusatz besteht aus einer U-förmigen, z.B. drittelrohrförmig ausgebildeten Schiene 38, welche in entsprechende, antero-posterior gelegene Kerben 39 im Prothesenschaft 1 über der Kammer 4 einsetzbar ist. Diese Schienen 38, welche mit ihrer Oeffnung zum Knochen 3 hin orientiert sind, erlauben den raschen Wiederaufbau einer medullären Vaskularität.

## Patentansprüche

1. Schaft für eine in einem Knochen (3) zu verankernde Endoprothese mit einer den Schaft (1) vollständig oder teilweise umgebenden, mit einem Füllmittel (16;17) expandierbaren, druckdicht verschliessbaren Kammer (4,4bis;18,19,20,21;18,19,20,21,28), dadurch gekennzeichnet, dass die Kammer (4,4bis;18,19,20,21;18,19,20,21,28) mit einem Einwegventil (9) versehen und mehrteilig ausgebildet ist.

2. Schaft nach Anspruch 1, gekennzeichnet durch eine erste, schaftnahe Kammer (4bis), welche von einer zweiten, knochennahen Kammer (4) umgeben ist.

3. Schaft nach Anspruch 1, gekennzeichnet durch vier Kammern, wobei eine proximo-mediale (18) mit einer latero-distalen (19) Kammer und eine proximo-laterale (20) mit einer medio-distalen (21) Kammer kommuniziert.

4. Schaft nach einem der Ansprüche 1 bis 3, gekennzeichnet durch eine im Inneren des Schaftes (1) gelegene mit der Kammer (4) kommunizierende Vorratskammer (29) für das Füllmaterial (16;17).

5. Schaft nach einem der Ansprüche 1 bis 4, dadurch gekennzeichnet, dass die Wandung der Kammer (4) aus Silikon oder Polyurethan besteht.

6. Schaft nach einem der Ansprüche 1 bis 4, dadurch gekennzeichnet, dass die Wandung der Kammer (4) aus einem, vorzugsweise mit Silikon oder Polyurethan imprägnierten, Geflecht besteht.

7. Schaft nach Anspruch 6, dadurch gekennzeichnet, dass das Geflecht aus zwei gegeneinander gekreuzten Serien von Fasern (42) besteht, welche vorzugsweise eine Neigung von etwa 45° zur Schaftachse (43) aufweisen.

8. Schaft nach Anspruch 7, dadurch gekennzeichnet, dass die Fasern (42) des Geflechtes aus Kohlenstoff, Polyester oder Polyamid bestehen.

9. Schaft nach einem der Ansprüche 1 bis 8, dadurch gekennzeichnet, dass die Wandstärke der Kammer (4) kleiner als 0,5 mm, vorzugsweise kleiner als 0,25 mm ist.

10. Schaft nach einem der Ansprüche 1 bis 9, dadurch gekennzeichnet, dass die Wandung der Kammer (4) im proximalen Bereich (41) und distalen Bereich (7) zugsteifer als im zentralen Bereich ausgebildet ist.

11. Schaft nach einem der Ansprüche 1 bis 10, dadurch gekennzeichnet, dass die Aussenseite der Kammer (4) mit Hydroxyapatit- oder Karbon-Partikeln beschichtet ist.

12. Schaft nach einem der Ansprüche 1 bis 11, gekennzeichnet durch proximo-distal verlaufende, zur Aufnahme von Metallschienen-Einsätzen (38) bestimmte, rinnenförmige Vertiefungen (39), vorzugsweise auf der anterioren und posterioren Seitenfläche des Prothesenschaftes (1).

13. Schaft nach einem der Ansprüche 1 bis 12, dadurch gekennzeichnet, dass die Kammer (4) und/oder eine mit der Kammer (4) kommunizierende Vorratskammer (29) permanent unter Druck steht.

14. Schaft nach Anspruch 13, dadurch gekennzeichnet, dass die Kammer (4) und/oder eine mit der Kammer (4) kommunizierende Vorratskammer (29) ein unter Druck stehendes thermoplastisches Füllmaterial (17) enthält.

15. Schaft nach Anspruch 13, dadurch gekennzeichnet, dass das thermoplastische Füllmaterial (17) durch die Kraft einer Feder (31) unter ständigem Druck steht.

16. Schaft nach einem der Ansprüche 1 bis 15, dadurch gekennzeichnet, dass die Kammer (4) derart am Prothesenschaft (1) befestigt ist, dass der Schaft (1) im implantierten Zustand unter Beibehaltung einer bewegungsfreien Kammer/Knochen-Anlage in beschränktem Umfange axial bewegbar ist.

17. Schaft nach einem der Ansprüche 1 bis 16, dadurch gekennzeichnet, dass die Kammer (4) mindestens an der dem Knochen (3) zugewandten Oberfläche aus einem bioaffinen Material besteht.

18. Schaft nach einem der Ansprüche 1 bis 17, dadurch gekennzeichnet, dass die Kammer (4) mindestens an der dem Knochen (3) zugewandten Oberfläche mit einem samtartigen Belag aus Kohlenstoffmaterial versehen ist.

19. Schaft nach einem der Ansprüche 1 bis 18, dadurch gekennzeichnet, dass das Füllmaterial (16) aus einer viskosen Flüssigkeit besteht.

20. Schaft nach Anspruch 19, dadurch gekennzeichnet, dass das Füllmaterial (16) aus einer mit pulver- oder faserförmigen Füllstoffpartikeln, vorzugsweise Kohlenstoffasern verdickten Flüssigkeit besteht.

21. Endoprothese mit einem Schaft nach einem der Ansprüche 1 bis 20.

22. Endoprothese nach Anspruch 21, dadurch gekennzeichnet, dass der Schaft als Verankerungselement für die Femurkomponente und/oder für die Pfannenkomponente einer Gelenkendoprothese ausgebildet ist.

## Claims

1. A stem for an endoprosthesis to be anchored in a bone (3) having a pressure-tight sealable chamber (4,4bis;18,19,20,21; 18,19,20,21,28) enclosing said stem (1) completely or partially and being expandable with a filler (16;17), characterized in that said chamber (4,4bis;18,19,20,21;18,19,20,21,28) is provided with a single-way cock (9) and is designed in multiple parts.

2. Stem according to claim 1, characterized by a first chamber (4bis) adjacent the stem and enclosed by a second chamber (4) adjacent the bone.

3. Stem according to claim 1, characterized by four chambers, where a proximo-medial chamber (18) communicates with a lateral-distal chamber (19) and a proximo-lateral chamber (20) communicates with a medio-distal chamber (21).

4. Stem according to one of the claims 1 to 3, characterized by a reservoir (29) for a filler (16;17) inside the stem (1) and communicating with the chamber (4).

5. Stem according to one of the claims 1 to 4, characterized in that the wall of the chamber (4) is made of silicone or polyurethane.

6. Stem according to one of the claims 1 to 4, characterized in that the wall of the chamber (4) consists of a braid impregnated preferably with silicone or polyurethane.

7. Stem according to claim 6, characterized in that the braid consists of two mutually crossing series of fibers (42) evincing a slope of about 45 relative to the stem axis (43).

8. Stem according to claim 7, characterized in that the braid fibers (42) consist of carbon, polyester or polyamide.

9. Stem according to one of the claims 1 to 8, characterized in that the wall thickness of the chamber (4) is less than 0,5 mm and preferably less than 0,25 mm.

10. Stem according to one of the claims 1 to 9, characterized in that the wall of the chamber (4) evinces more tensile strength in the proximal region (41) and in the distal region (7) than in the central region.

11. Stem according to one of the claims 1 to 10, characterized in that the outside of the chamber (4) is coated with hydroxy-apatite particles or with carbon particles.

12. Stem according to one of the claims 1 to 11, characterized by gutter-like notches (39) to receive metal rail-like insets (38) and extending in the proximo-distal direction, preferably being present on the anterior and posterior sides of the prosthesis stem (1).

13. Stem according to one of the claims 1 to 12, characterized in that the chamber (4) and/or a supply chamber (29) communicating with the chamber (4) is permanently pressurized.

14. Stem according to claim 13, characterized in that the chamber (4) and/or a supply chamber (29) communicating with chamber (4) contains a pressurized, thermoplastic filler (17).

15. Stem according to claim 13, characterized in that the thermoplastic filler (17) is permanently pressurized by the force exerted by a spring (31).

16. Stem according to one of the claims 1 to 15, characterized in that the chamber (4) is affixed in such manner to the prosthesis stem (1) that the stem (1) when implanted is axially displaceable in a limited way while retaining immobile rest between the chamber and the bone.

17. Stem according to one of the claims 1 to 16, characterized in that the chamber (4) consists, at least at the surface facing the bone (3), of a biologically affinitive material.

18. Stem according to one of the claims 1 to 17, characterized in that the chamber (4) is provided, at least at the surface facing the bone (3), with a carbonaceous velvet-like layer.

19. Stem according to one of the claims 1 to 18, characterized in that the filler (16) consists of a viscous liquid.

20. Stem according to claim 19, characterized in that the filler (16) consists of a liquid thickened with filler particles of powder or fibers, preferably carbon fibers.

21. Endoprosthesis with a stem according to one of claims 1 to 20.

22. Endoprosthesis according to claim 21, characterized in that the stem is designed as an anchoring element for the femoral component and/or for the socket component of a joint-endoprosthesis.

## Revendications

1. Tige pour une endoprothèse à ancrer dans un os (3), comportant une chambre (4, 4bis; 18, 19, 20, 21; 18, 19, 20, 21, 28) entourant complètement ou partiellement la tige (1), pouvant être expansée avec un agent de remplissage (16; 17), pouvant être refermée de manière hermétique sous pression, caractérisée en ce que la chambre (4, 4bis; 18, 19, 20, 21; 18, 19, 20, 21, 28) est dotée d'un clapet antiretour (9) et est réalisée en plusieurs parties.

2. Tige selon la revendication 1, caractérisée par une première chambre (4bis) proche de la tige, et qui est entourée par une seconde chambre (4) proche de l'os.

3. Tige selon la revendication 1, caractérisée par quatre chambres, une chambre proximo-médiale (18) communiquant avec une chambre latéro-distale (19), et une chambre latéro-proximale (20) communiquant avec une chambre médio-distale (21).

4. Tige selon l'une des revendications 1 à 3, caractérisée par une chambre de réserve (29) pour le matériau de remplissage (16; 17) située à l'intérieur de la tige (1) et communiquant avec la chambre (4).

5. Chambre selon l'une des revendications 1 à 4, caractérisée en ce que la paroi de la chambre (4) est constituée de silicone ou de polyuréthane.

6. Tige selon l'une des revendications 1 à 4, caractérisée en ce que la paroi de la chambre (4) est constituée d'un treillis, de préférence imprégné de silicone ou de polyuréthane.

7. Tige selon la revendication 6, caractérisée en ce que le treillis est constitué de deux séries de fibres (42) mutuellement croisées, qui présentent de préférence une inclinaison d'environ 45° par rapport à l'axe (43) de la tige.

8. Tige selon la revendication 7, caractérisée en ce que les fibres (42) du treillis sont constituées de carbone, de polyester ou de polyamide.

9. Tige selon l'une des revendications 1 à 8, caractérisée en ce que l'épaisseur de la paroi de la chambre (4) est inférieure à 0,5 mm, de préférence inférieure à 0,25 mm.

10. Tige selon l'une des revendications 1 à 9, caractérisée en ce que la paroi de la chambre (4) est dans la zone proximale (41) et dans la zone distale (7) configurée de manière plus résistante à la traction que dans la zone centrale.

11. Tige selon l'une des revendications 1 à 10, caractérisée en ce que la face extérieure de la chambre (4) est revêtue de particules d'hydroxyapatite ou de carbone.

12. Tige selon l'une des revendications 1 à 11, caractérisée par des renfoncements (38), en forme de rigoles s'étendant dans la direction proximo-distale, destinés à reprendre des garnitures en rails métalliques (39), de préférence sur les faces latérales antérieure et postérieure de la tige (1) de la prothèse.

13. Tige selon l'une des revendications 1 à 12, caractérisée en ce que la chambre (4) et/ou une chambre de réserve (29) communiquant avec la chambre (4) sont maintenues en permanence sous pression.

14. Tige selon la revendication 13, caractérisée en ce que la chambre (4) et/ou une chambre de réserve (29) communiquant avec la chambre (4) contiennent un matériau de remplissage (17) thermoplastique maintenu sous pression.

15. Tige selon la revendication 13, caractérisée en ce que le matériau thermoplastique (17) est maintenu sous pression permanente par la force d'un ressort (31).

16. Tige selon l'une des revendications 1 à 15, caractérisée en ce que la chambre (4) est fixée à la tige (1) de la prothèse, de telle sorte que dans l'état implanté, la tige (1) est déplaçable axialement sur une plage limitée tout en maintenant une pose sans déplacement de la chambre sur l'os.

17. Tige selon l'une des revendications 1 à 16, caractérisée en ce que la chambre (4) est au moins sur sa surface tournée vers l'os (3) constituée d'un matériau bioaffine.

18. Tige selon l'une des revendications 1 à 17, caractérisée en ce que la chambre (4) est dotée au moins sur la surface tournée vers l'os (3) d'un revêtement velouté en matériau de carbone.

19. Tige selon l'une des revendications 1 à 18, caractérisée en ce que le matériau de remplissage (16) est constitué d'un liquide visqueux.

20. Tige selon la revendication 19, caractérisée en ce que le matériau de remplissage (16) est constitué d'un liquide épaissi avec des particules de remplissage pulvérulentes ou fibreuses, de préférence des fibres de carbone.

21. Endoprothèse avec tige selon l'une des revendications 1 à 20.

22. Endoprothèse selon la revendication 21, caractérisée en ce que la tige est configurée comme élément d'ancrage pour le composant de fémur et/ou pour le composant de cavité d'une endoprothèse articulaire.
